# DEMANDE DE BREVET EUROPEEN

(11) **EP 1 062 956 A1**
(43) Date de publication de la demande: **27.12.2000**
(21) Numéro de dépôt: 00420132.3
(22) Date de dépôt: 21.06.2000
(51) Int. Cl.: A61L 27/56, A61L 27/32, A61L 27/50

(54) **Matériel prothétique ou d'ostéosynthèse et procédé de fabrication d'un tel matériel**

(30) Priorité: 23.06.1999 FR 9908196
(71) Demandeur: Nesme, Marie Corinne, épose Gauthier, 69006 Lyon (FR)
(72) Inventeur: Nesme, Marie Corinne, épose Gauthier, 69006 Lyon (FR)
(74) Mandataire: Myon, Gérard Jean-Pierre

(57) **Abrégé**

Ce matériel (1) comprend une pièce rigide (2), notamment métallique, au moins partiellement revêtue d'une couche (3) d'un matériau bio-réactif, notamment à base d'hydroxyapatite de calcium ou de phosphate tricalcique. La surface externe (3a) de cette couche (3) est lissée, de telle sorte qu'elle ne présente pas d'aspérité (3b) s'étendant au-delà d'une surface d'enveloppe (S). Cette surface externe (3a) est pourvue de micro-cavités (3c) formées en deçà de la surface d'enveloppe (S).

## Description

L'invention a trait à un matériel prothétique ou d'ostéosynthèse comprenant une pièce rigide, notamment métallique, revêtue d'une couche d'un matériau bio-réactif, ainsi qu'à un procédé de fabrication d'un tel matériel.

Dans le domaine des prothèses, en particulier celui des prothèses de hanche, il est connu depuis longtemps de revêtir la queue d'une prothèse fémorale, habituellement en alliage de titane, d'une couche bio-réactive, les matériaux le plus couramment utilisés étant de l'hydroxyapatite de calcium ou un mélange hydroxyapatite-phosphate tricalcique. En effet, la couverture du matériel métallique par le matériau bio-actif permet un ancrage rapide et fiable de la prothèse sur l'os.

Par ailleurs, il est connu, notamment de FR-A-2 706 280 ou de EP-A-0 532 421, de réaliser un matériel d'ostéosynthèse à partir d'une pièce métallique revêtue d'un matériau biocompatible, tel que de l'hydroxyapatite de calcium ou un phosphate de calcium. L'utilisation d'un tel matériau biocompatible permet un ancrage mécanique rapide entre le matériel et les différents éléments osseux à solidariser.

Après la fusion osseuse, une fixation os/matériel d'ostéosynthèse très stable entraîne les inconvénients suivants : le "shunt mécanique" ainsi obtenu perturbe la structure osseuse ; on a parlé de spongialisation. De plus, si l'ablation du matériel d'ostéosynthèse doit être effectuée, la difficulté de le retirer sans lésion osseuse est importante.

Dans le domaine des prothèses, par exemple en ce qui concerne la queue fémorale des prothèses totales de hanche, il est admis que la partie proximale de la queue doit être solidement fixée à l'os spongieux de l'extrémité supérieure du fémur. Il a été démontré qu'une fixation rigide de la partie distale de la queue sur l'os cortical diaphysaire entraîne des mouvements secondaires et tardifs.

Parmi les inconvénients secondaires, on cite des réactions douloureuses et hypertrophiques au niveau de l'extrémité de la queue. Parmi les inconvénients tardifs, on cite l'ostéolyse fémorale proximale, en particulier de l'éperon de Merkel.

Selon une technique classique, il est connu d'appliquer l'hydroxyapatite de calcium au moyen d'une torche à plasma. La surface extérieure de la couche d'hydroxyapatite ainsi déposée est irrégulière, ce qui ne pose pas de problème lors de la mise en place d'un élément prothétique par coincement de deux surfaces de portées tronconiques ou évasées l'une contre l'autre. De même, une plaque d'ostéosynthèse recouverte d'hydroxyapatite rugueuse s'applique facilement sur la surface extérieure d'un os.

En revanche, dans le cas d'un matériel de forme globalement cylindrique ou parallélépipédique devant être introduit dans un logement ou canal globalement cylindrique ou parallélépipédique, les forces de frottement entre la surface externe de la couche de matériau bio-réactif et la surface interne du canal ou logement sont telles qu'il se produit, par cisaillement, un arrachement ou une désolidarisation de cette couche de matériau bio-réactif par rapport à la pièce rigide. Il n'est alors plus possible d'obtenir un ancrage rapide du matériel par rapport à la structure osseuse avoisinante, le matériau bio-réactif étant alors inefficace. Si les dimensions intérieures du logement sont augmentées pour éviter cet arrachement, le matériau bio-réactif n'est plus en contact serré avec l'os, et ne joue plus son rôle : il n'y a plus d'ancrage possible.

C'est à ces inconvénients qu'entend plus particulièrement remédier l'invention en proposant un matériel susceptible d'être introduit dans un logement, de forme correspondant à celle de ce matériel, sans arrachement de la couche de matériau bio-réactif par rapport à la pièce rigide.

Dans cet esprit, l'invention concerne un matériel prothétique ou d'ostéosynthèse comprenant une pièce rigide, notamment métallique, au moins partiellement revêtue d'une couche de matériau bio-réactif, notamment à base d'hydroxyapatite de calcium ou de phosphate tricalcique, caractérisé en ce que la surface externe de cette couche est au moins partiellement lissée, de telle sorte qu'elle ne présente pas d'aspérité s'étendant au-delà d'une surface d'enveloppe, et en ce que cette surface externe est pourvue de micro-cavités formées en deçà de cette surface d'enveloppe.

Grâce à l'invention, le lissage de la surface externe de la couche de matériau bio-réactif évite que des aspérités n'interagissent avec les os voisins, au point de générer des frottements susceptibles de désolidariser la couche de matériau bio-réactif par rapport à la pièce rigide lors du mouvement de pénétration du matériel dans l'os. Les micro-cavités qui demeurent après ce lissage permettent un ancrage efficace du matériau bio-réactif dans la structure osseuse voisine. En d'autres termes, le caractère lissé de la couche de matériau bio-réactif lui permet de ne pas gêner la mise en place du matériel, alors que les micro-cavités qu'elle comprend, à l'intérieur de la surface d'enveloppe, lui permettent d'accélérer la fixation du matériel dans la structure osseuse.

Selon des aspects avantageux de l'invention, le matériel incorpore une ou plusieurs des caractéristiques suivantes :
- les micro-cavités ont une longueur maximale inférieure à 400 micromètres, de préférence de l'ordre de 200 micromètres, avec une largeur minimale supérieure à 10 micromètres, de préférence de l'ordre de 20 micromètres.
- la surface de la pièce rigide revêtue de matériau bio-réactif est lisse. En particulier, les micro-cavités de cette surface ont des dimensions inférieures ou égales à celles mentionnées ci-dessus ;

Selon des variantes de réalisation de l'invention, la pièce rigide est globalement cylindrique ou au moins partiellement plane.

L'invention concerne également un procédé de fabrication d'un matériel tel que précédemment décrit et, plus précisément, un procédé comprenant une étape d'application d'une couche de matériau bio-réactif sur une pièce rigide, notamment métallique, caractérisé en ce qu'il consiste à faire subir un traitement de lissage, par usinage, écrétage ou moulage de ses aspérités, à une partie au moins de la surface externe de la couche de matériau bio-réactif en créant une surface d'enveloppe en deçà de laquelle des micro-cavités demeurent dans cette surface.

Selon un aspect avantageux de l'invention, le procédé consiste également à faire subir un traitement de lissage à la surface de la pièce rigide préalablement à l'application de la couche de matériau bio-réactif. Ceci permet de contrôler le niveau d'adhésion de la couche de matériau bio-réactif sur la pièce rigide. En particulier, on peut prévoir de laisser subsister, dans la surface extérieure de la pièce rigide, des micro-cavités de longueur et de largeur inférieures ou égales à celles des micro-cavités de la surface externe de la couche de matériau bio-réactif. Ces micro-cavités d'interface ont, de préférence, au moins une dimension inférieure à 20 micromètres, afin d'éviter ou de limiter la repousse osseuse après disparition de la couche bio-réactive.

L'invention concerne enfin un matériel prothétique ou d'ostéosynthèse fabriqué selon le procédé précédemment décrit.

L'invention sera mieux comprise et d'autres avantages de celle-ci apparaîtront plus clairement à la lumière de la description qui va suivre de deux modes de réalisation d'un matériel conforme à son principe, donnée uniquement à titre d'exemple et faite en référence aux dessins annexés dans lesquels :
- la figure 1 est une vue de côté d'une broche d'ostéosynthèse conforme à l'invention ;
- la figure 2 est une vue du détail II à la figure 1, à plus grande échelle, lors d'une première étape de fabrication de la broche de la figure 1 ;
- la figure 3 est une vue analogue à la figure 2 au terme de la fabrication de la broche ;
- la figure 4 est une coupe selon la ligne IV-IV à la figure 3 ;
- la figure 5 est une vue de côté d'une prothèse fémorale implantée, conforme à un second mode de réalisation de l'invention et
- la figure 6 est une coupe partielle à plus grande échelle selon la ligne VI-VI à la figure 5.

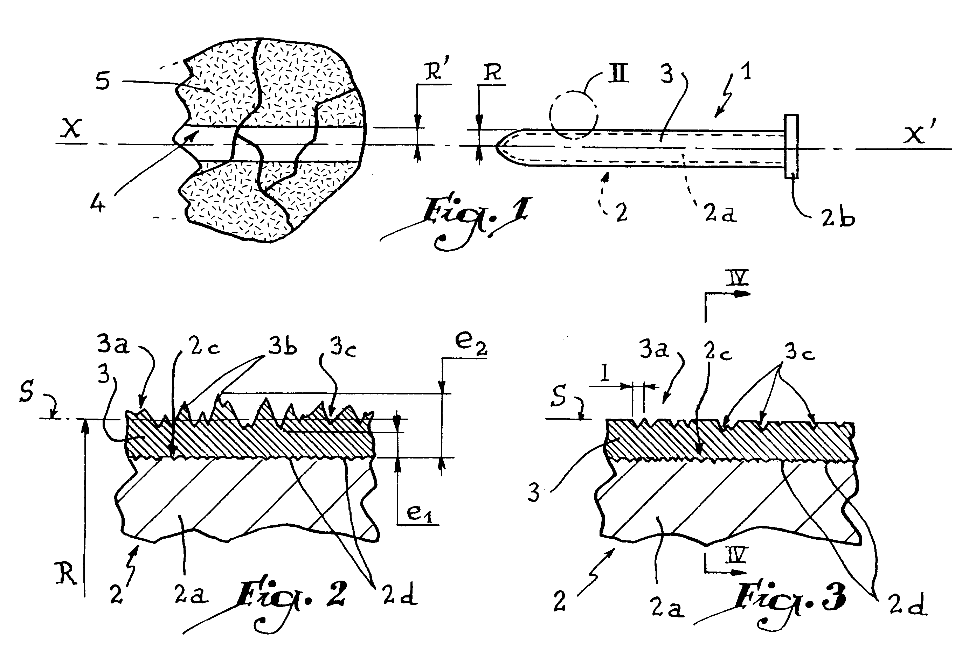

La broche 1 représentée à la figure 1 comprend une âme 2 réalisée en titane et formant une tige globalement cylindrique 2a et une tête 2b. La tige 2a est revêtue d'une couche 3 d'hydroxyapatite de calcium déposée au moyen d'une torche à plasma.

Comme il ressort plus clairement de la figure 2, après le dépôt de la couche 3, la surface extérieure 3a de celle-ci comprend des aspérités 3b et des micro-cavités 3c. Cette surface 3a est donc irrégulière.

En pratique, la couche 3 a une épaisseur comprise entre une valeur minimale e₁ de l'ordre de 50 micromètres et une valeur maximale e₂ de l'ordre de 200 micromètres.

Conformément à l'invention, la surface externe 3a de la couche 3 est lissée, de telle sorte qu'elle ne dépasse pas au-delà d'une surface cylindrique S centrée sur l'axe de X-X' de la tige 2a et dont on note R le rayon. Le lissage de la surface 3a peut être obtenu par usinage, notamment par meulage. Ainsi, les aspérités 3b sont écrêtées dans la mesure où leurs parties qui dépassent à l'extérieur de la surface S sont éliminées.

En revanche, les micro-cavités 3c demeurent dans leurs parties situées en deçà de la surface S par rapport à la tige 2a, c'est-à-dire à l'intérieur de l'enveloppe formée par cette surface. Ainsi, la surface 3a est calibrée à la côte de la surface S en laissant subsister les micro-cavités 3c.

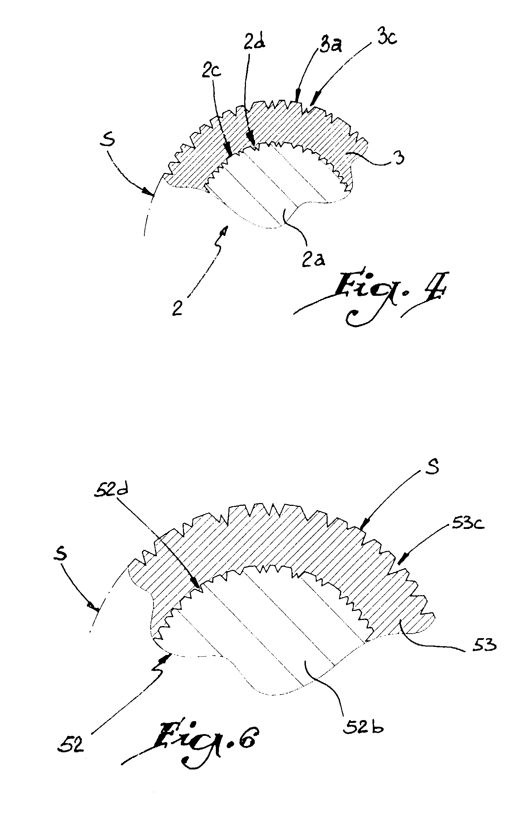

Au terme de l'opération d'usinage, la couche 3 présente le profil visible aux figures 3 et 4, dans lequel seules les micro-cavités 3c constituent des reliefs, en creux, de la surface 3a.

La longueur l des micro-cavités 3c est inférieure à 400 micromètres alors que leur largeur est supérieure à 10 micromètres, de telle sorte que leur surface totale est adaptée à un accrochage de la couche 3 avec l'os. On peut choisir la longueur des micro-cavités de l'ordre de 200 micromètres et leur largeur de l'ordre de 20 micromètres.

Les micro-cavités 3c occupent moins de 20% de la surface 3a, de sorte que les susrfaces 3a et S sont confondues sur leurs plus grandes parties.

Selon un aspect avantageux de l'invention, la surface externe 2c de la tige 2a est lissée avant application de la couche 3. En pratique, ce lissage, qui a lieu par usinage ou traitement physico-chimique de surface, laisse subsister des micro-cavités 2d de longueur et largeur inférieures ou égales à celles des micro-cavités 3c. On évite ainsi un ancrage secondaire rigide entre l'os et le métal, ce qui favorise le retrait de l'âme 2 au terme de l'ostéosynthèse. Grâce à l'invention, la faible force d'adhésion entre la couche 3 et la tige 2a n'est pas gênante lors de la mise en place, par exemple par impaction, de la broche 1 dans un logement cylindrique 4, de rayon R' sensiblement égal au rayon R, formé dans un amas osseux 5. En revanche, cette faible force d'adhérence permet l'ablation du matériel d'ostéosynthèse.

Les micro-cavités 2d sont réalisées en plus grand nombre que les micro-cavités 3c, alors qu'elles sont de dimensions inférieures. En pratique, ces micro-cavités 2d sont la trace d'irrégularités de surface de la tige 2a, ces irrégularités étant atténuées par le traitement de lissage. La tige 2a peut avoir un aspect extérieur "fendillé" ou "fibreux" qui provient de la nature de sa couche superficielle qui est en titane.

Le mode de fabrication et de lissage de la tige 2 est choisi de telle sorte que la largeur des microvatités 2d soit inférieure à 20 micromètres afin d'éviter ou de limiter une repousse osseuse dans la tige après disparition de la couche 3. Ceci autorise le retrait ultérieur de la broche 1 sans destruction de l'os.

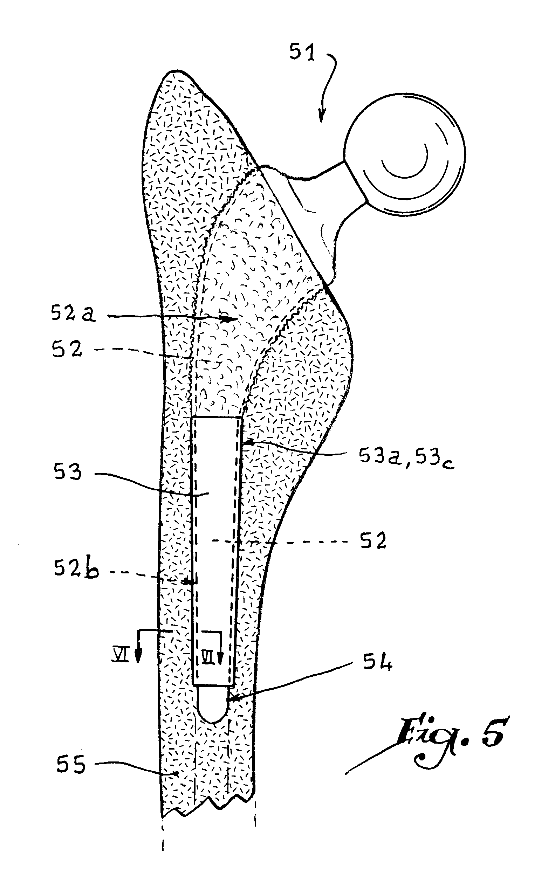

Dans le second mode de réalisation de l'invention représenté à la figure 5, une prothèse fémorale 51 comprend une queue 52 destinée à être introduite dans le canal médullaire 54 d'un fémur 55. Une couche 53 de phosphate tricalcique est appliquée, sur toute la surface de la queue 52. Cette couche est conservée irrégulière sur la portion proximale évasée 52a de la queue 52, conformément aux habitudes du métier. Par contre, et conformément à l'invention, autour d'une partie distale cylindrique 52b de la queue 52, la surface extérieure 53a de cette couche 53 est lissée par un procédé mécanique, notamment par meulage ou usinage, afin d'évacuer les aspérités résultant du procédé d'application du phosphate tricalcique sur la partie 52a de la queue 52. La surface 53a est rectifiée selon la géométrie d'une surface d'enveloppe S qui a, dans l'exemple, la forme d'un cylindre à base circulaire. Des micro-cavités 53c demeurent sur la surface 53a, en deça de la surface S, et permettent un ancrage rapide de la prothèse 51 dans le fémur. Les micro-cavités ont des dimensions du même ordre de grandeur que les cavités 3c du premier mode de réalisation. Comme précédemment elles occupent moins de 20% de la surface 53a.

Comme précédemment, la surface extérieure 52c de la partie 52b de la queue 52 est elle-même lissée avant application de la couche 53. Des micro-cavités 52d sont formées, en plus grand nombre et avec des dimensions inférieures à celles des micro-cavités 53c, sur la surface 52c. Ces micro-cavités ont une largeur inférieure à 20 micromètres.

Comme les micro-cavités 52d de cette surface 52c ont des dimensions inférieures ou égales à celles des cavités 53c, la mise en place de la partie distale de la queue 52 est possible, sans arrachement de la couche 53, et permet un ancrage entre l'os et le matériel bio-réactif. Secondairement et tardivement, le lissage du métal évite un ancrage rigide définitif os/métal après disparition au moins partielle de la couche 53. Les problèmes de shunt mécanique de l'état de la technique ne se posent alors pas.

Selon une variante de réalisation non représentée de l'invention, la surface de la partie rigide de l'élément prothétique ou d'ostéosynthèse sur laquelle est appliquée la couche du matériau bio-réactif peut être globalement plane, voire prendre toute forme adaptée à la fonction requise. La forme de la surface d'enveloppe, c'est-à-dire de la surface extérieure finale de la couche de matériau bio-réactif est adaptée en conséquence.

Quel que soit le mode de réalisation considéré, le fait que la surface externe de la couche d'hydroxyapatite de calcium ou d'un matériau équivalent est calibrée et lissée permet une mise en place aisée du matériel prothétique ou d'ostéosynthèse, sans danger d'arrachement de cette couche, alors que le caractère lisse de la surface de la pièce rigide évite des contraintes excessives durables entre la pièce rigide et l'os, notamment lors du retrait d'un matériau d'ostéosynthèse ou afin d'éviter qu'un schunt mécanique soit réalisé par une partie de la pièce rigide dans le cas d'une prothèse.

L'invention est applicable quel que soit le mode d'application de la couche de matériau bio-réactif sur la pièce rigide. L'invention est applicable indépendamment de la nature exacte du matériau constitutif de la pièce rigide. Elle trouve cependant une application particulièrement importante avec des pièces métalliques, notamment réalisées en titane ou dans un alliage à base de titane.

## Revendications

1. Matériel prothétique (51) ou d'ostéosynthèse (1) comprenant une pièce rigide (2 ; 52), notamment métallique, au moins partiellement revêtue d'une couche (3 ; 53) d'un matériau bio-réactif, notamment à base d'hydroxyapatite de calcium ou de phosphate tricalcique, caractérisé en ce que la surface externe (3a ; 53a) de ladite couche est au moins partiellement lissée, de telle sorte qu'elle ne présente pas d'aspérité (3b) s'étendant au-delà d'une surface d'enveloppe (S), et en ce que ladite surface externe est pourvue de micro-cavités (3c ; 53c) formées en deçà de ladite surface d'enveloppe.

2. Matériel selon la revendication 1, caractérisé en ce que lesdites micro-cavités (3c ; 53c) ont une longueur maximale (l) inférieure à 400 micromètres, de préférence de l'ordre de 200 micromètres, avec une largeur minimale supérieure à 10 micromètres, de préférence de l'ordre de 20 micromètres.

3. Matériel selon l'une des revendications 1 ou 2, caractérisé en ce que la surface (2c ; 52c) de ladite pièce rigide (2 ; 52) revêtue de la couche (3 ; 53) de matériau bio-réactif est lisse.

4. Matériel selon la revendication 3, caractérisé en ce que ladite surface (2c ; 52c) de ladite pièce rigide (2 ; 52) a des micro-cavités (2d ; 52d) de longueur et largeur inférieures ou égales à celles des micro-cavités (3c ; 53c) de la surface externe (3a ; 53a) de la couche (3 ; 53) de matériau bio-réactif.

5. Matériel selon l'une des revendications précédentes, caractérisé en ce que ladite pièce rigide (2 ; 52) est globalement cylindrique.

6. Matériel selon l'une des revendications précédentes, caractérisé en ce que ladite pièce rigide est au moins partiellement plane.

7. Procédé de fabrication d'un matériel prothétique (51) ou d'ostéosynthèse (1) comprenant une étape d'application d'une couche (3 ; 53) de matériau bio-réactif sur une pièce rigide (2 ; 52), notamment métallique, caractérisé en ce qu'il consiste à faire subir un traitement de lissage, par usinage, écrêtage ou meulage de ses aspérités (3b), à une partie au moins de la surface externe (3a ; 53a) de ladite couche de matériau bio-réactif en créant une surface d'enveloppe (S) en deçà de laquelle des micro-cavités (3c ; 53c) demeurent dans ladite surface.

8. Procédé selon la revendication 7, caractérisé en ce qu'il consiste à faire subir un traitement de lissage à la surface (2c ; 52c) de ladite pièce rigide (2 ; 52) préalablement à l'application de ladite couche de matériau bio-réactif.

9. Procédé selon la revendication 8, caractérisé en ce que ledit traitement de lissage laisse subsister, dans ladite surface (2c ; 52c) de ladite pièce rigide (2 ; 52) des micro-cavités (2d ; 52d) de longueur et largeur inférieures ou égales à celles des micro-cavités (3c, 53c) de la surface externe (3a ; 53a) de ladite couche (3 ; 53) de matériau bio-réactif.

10. Matériel selon la revendication 4 ou procédé selon la revendication 9, caractérisé en ce que lesdites micro-cavités (2d ; 52d) de ladite surface (2c ; 52c) de ladite pièce rigide (2 ; 52) ont au moins une dimension inférieure à 20 micromètres.
